# EUROPEAN PATENT SPECIFICATION

(11) **EP 0 470 711 B1**
(45) Date of publication and mention of the grant of the patent: **17.05.1995**
(21) Application number: 91306447.3
(22) Date of filing: 16.07.1991
(51) Int. Cl.: C07C 237/42, A01N 37/46, A01N 53/00, C07C 247/04, C07C 255/03

(54) **Fungicides**
Fungizide
Composés fongicides

(30) Priority: 27.07.1990 GB 9016582
(43) Date of publication of application: 12.02.1992
(73) Proprietor: ZENECA LIMITED, London W1Y 6LN (GB)
(72) Inventor: Crowley, Patrick Jelf, Crowthorne, Berkshire (GB); Glen, Alasdair Thomas, Macclesfield, Cheshire SK10 2HQ (GB); Spence, Rosamund Alison, Caversham, Berkshire RG4 7TQ (GB); Lawson, Kevin Robert, Piddington, High Wycombe, Bucks HP14 3BB (GB)
(74) Representative: Tierney, Francis John

(56) References cited:
- EP-A- 0 100 615
- EP-A- 0 127 990
- EP-A- 0 381 330

## Description

This invention relates to novel fungicidal acylaminobenzamides, to processes for preparing them, to fungicidal compositions containing them and to methods of using them to combat fungi, especially fungal infections of plants.

Acknowledgement is made of UK Application No. 42454/77 from which US-A-4282218, for example, claims priority and of EP-A-0127990. The former describes acylanilides which have antiandrogenic properties and the latter describes aniline derivatives which have fungicidal properties.

According to the present invention there is provided a compound of the formula (I): 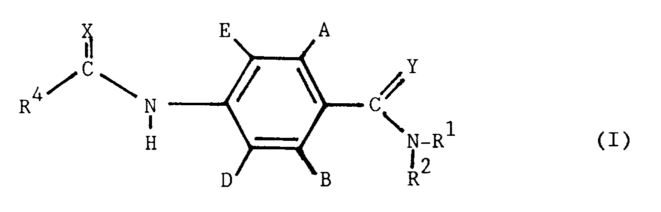
in which A and B are independently H, fluoro, chloro, bromo, iodo, C₁₋₄ alkyl, C₁₋₄ alkoxy, halo(C₁₋₄)alkyl, cyano, C₁₋₄ alkoxycarbonyl, C₁₋₄ alkoxymethyl, C₁₋₄ alkylthiomethyl, C₂₋₄ alkenyl, C₂₋₄ alkynyl, formyl, C₁₋₄ alkylthio, carboxy, C₁₋₄ alkylcarbonyl or nitro, provided that both A and B are not H; D and E are independently H or fluoro; R¹ and R² are independently C₁₋₄ alkyl, C₃₋₆ alkenyl (optionally substituted with halogen), C₃₋₆ alkynyl (optionally substituted with halogen), C₁₋₄ alkoxy, C₁₋₄ haloalkyl, C₃₋₆ cycloalkyl, C₃₋₆ cycloalkyl(C₁₋₄)alkyl, C₁₋₄ alkoxy(C₁₋₄)alkyl, C₁₋₄ alkylthio(C₁₋₄)alkyl or cyano; or R¹ and R² together with the nitrogen atom to which they are attached join to form a morpholine, piperidine, pyrrolidine or azetidine ring which is optionally substituted with C₁₋₄ alkyl; R⁴ is cyclobutyl, cyclopentyl or cyclohexyl, any of which is optionally substituted with halogen, C₁₋₄ alkyl, C₁₋₄ alkoxy, C₁₋₄ alkylthio, C₂₋₄ alkenyl, C₂₋₄ alkynyl, cyano, C₁₋₄ alkylcarbonyl, C₁₋₄ alkoxycarbonyl, azido, nitro, isocyano or NR⁵R⁶ (where R⁵ and R⁶ are independently H, C₁₋₄ alkyl, C₁₋₄ alkylcarbonyl or formyl) or R⁴ is C₂₋₈ alkyl substituted with azido, nitro, isocyano, -NR⁵R⁶ (where R⁵ and R⁶ are independently C₁₋₄ alkyl, C₁₋₄ alkylcarbonyl or formyl), cyano, C₁₋₄ alkylcarbonyl or C₁₋₄ alkoxycarbonyl; and X and Y are independently oxygen or sulphur.

Alkyl groups and the alkyl moiety of other alkyl-containing groups can be in the form of straight or branched chains. Examples are methyl, ethyl, propyl (n-and iso-propyl), butyl (n-, sec, iso- and t-butyl), 1,1-dimethylpropyl and 1,1-dimethylbutyl. Alkenyl and alkynyl groups can also be in the form of straight or branched chains. Examples are 1,1-dimethylbut-3-enyl and 1,1-dimethylprop-2-ynyl.

Halogen includes fluorine, chlorine and bromine.

In one aspect the invention provides a compound of formula (I) in which A is fluoro, chloro, bromo, iodo, C₁₋₄ alkyl, halo(C₁₋₄)alkyl, C₁₋₄ alkoxy, C₁₋₄ alkylthio, cyano, nitro, C₁₋₄ alkoxycarbonyl, C₁₋₄ alkoxy(C₁₋₄)alkyl, C₁₋₄ alkylthio(C₁₋₄)alkyl, C₂₋₄ alkenyl, C₂₋₄ alkynyl or C₁₋₄ alkylcarbonyl; B is H; D and E are independently H or fluoro; R¹ is methyl; R² is C₁₋₄ alkyl, halo(C₁₋₄)alkyl, C₃₋₄ alkenyl, C₃₋₄ alkynyl, cyclopropyl, cyclopropyl(C₁₋₄)alkyl, C₁₋₄ alkoxy, C₁₋₄ alkoxy(C₁₋₄)alkyl, C₁₋₄ alkylthio(C₁₋₄)alkyl or cyano; R⁴ is cyclobutyl, cyclopentyl or cyclohexyl (each optionally substituted in the 1-position with halogen, C₁₋₄ alkyl or C₁₋₄ alkoxy), or the group R³(CH₃)₂C- in which R³ is azido, nitro, cyano, NR⁵R⁶ (where R⁵ and R⁶ are independently H, C₁₋₄ alkyl or formyl), C₁₋₄ alkoxycarbonyl or C₁₋₄ alkylcarbonyl; and X and Y are both O.

In another aspect the invention provides a compound of formula (I) in which A is fluoro, chloro, bromo, iodo or methyl; B is H, fluoro or chloro; D and E are both H; R¹ and R² are both methyl; R⁴ is cyclobutyl, cyclopentyl or cyclohexyl (each optionally substituted in the 1-postion with halogen, C₁₋₄ alkyl or C₁₋₄ alkoxy), or the group R³(CH₃)₂C- in which R³ is azido, nitro, cyano, NR⁵R⁶ (where R⁵ and R⁶ are independently H, C₁₋₄ alkyl or formyl), C₁₋₄ alkoxycarbonyl or C₁₋₄ alkylcarbonyl; and X and Y are both oxygen.

In yet another aspect the invention provides a compound of formula (I) in which A is fluoro, chloro, bromo, iodo or methyl; B is H, fluoro or chloro; D and E are both H; R¹ and R² are both methyl; R⁴ is cyclobutyl, cyclopentyl or cyclohexyl (each substituted in the 1-position with methyl or fluoro); and X and Y are both oxygen.

In still yet another aspect the invention provides a compound of formula (I) in which A is fluoro, chloro, bromo, iodo or methyl; B is H, fluoro or chloro; D and E are both H; R¹ and R² are both methyl; R⁴ is 1-methylcyclobutyl, 1-methylcyclopentyl or 1-methylcyclohexyl; and X and Y are both oxygen.

The invention is illustrated by the compounds listed in Table I which follows. The compounds have the general formula (I.1) in which R¹, R², R⁴ A and B have the values listed in the table.

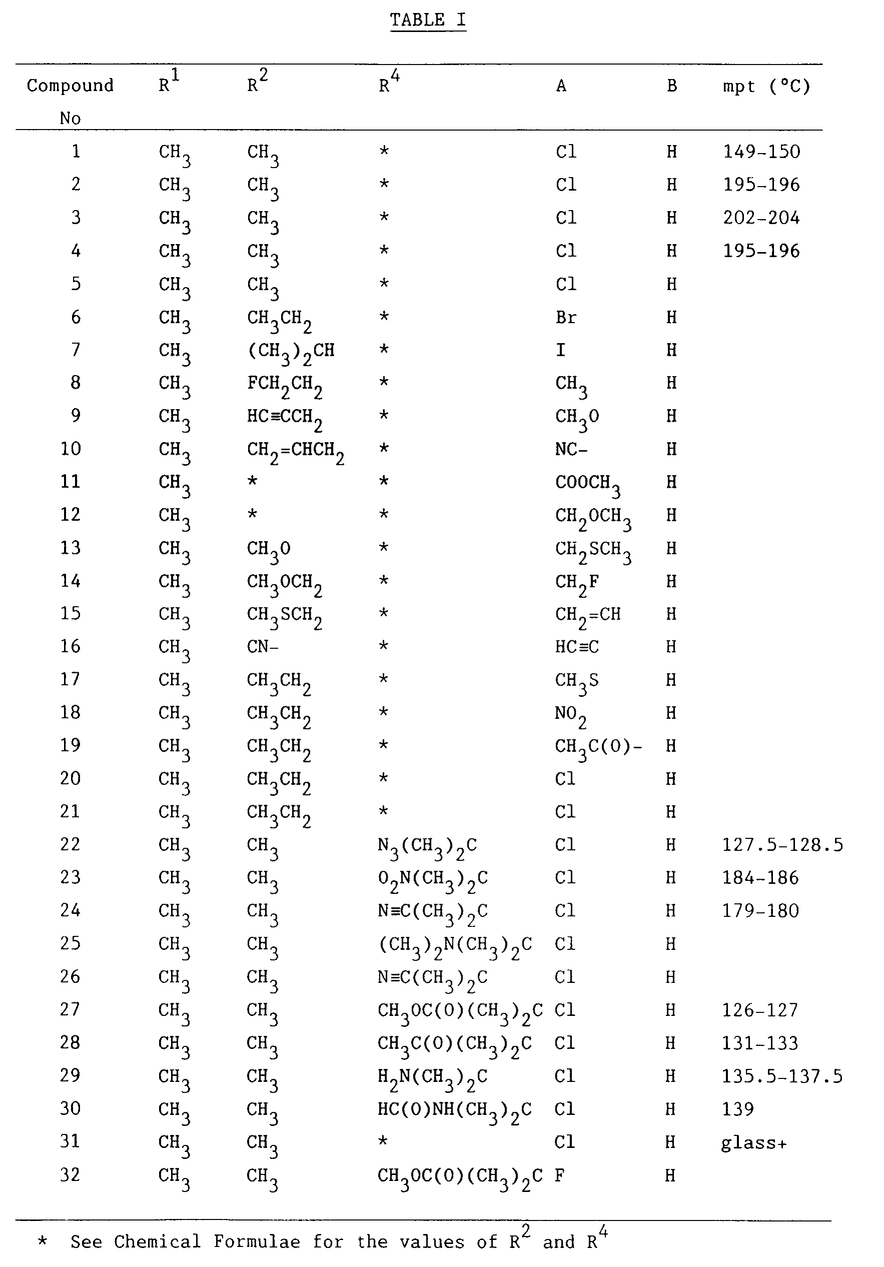

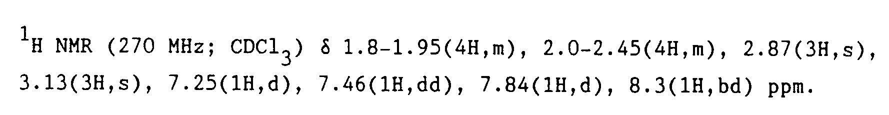

The compounds of the invention can be made by, for example, the methods illustrated in Schemes 1, 2, 3, 4 and 5. Throughout these Schemes R¹, R², R⁴, A, B, D, and E are as defined before.

In Scheme 1, compounds of formula (II) can be prepared by reacting compounds of formula (III) with an acid chloride R⁴COCl in a suitable organic solvent such as methylene chloride or toluene in the presence of a base such as a tertiary amine (for example triethylamine) or an alkali metal carbonate or hydroxide (for example sodium bicarbonate or sodium hydroxide).

In Scheme 2, compounds of formula (II) can be prepared from compounds of formula (VI) by reaction with an amine R¹R²NH in a suitable organic solvent such as methylene chloride or tetrahydrofuran (THF) in the presence of a base such as triethylamine, sodium bicarbonate or excess R¹R²NH.

Scheme 3 shows how compounds of formula (II) may be prepared when R⁴ is C₂₋₈ alkyl containing an azido substituent or a substituent -NR⁵R⁶ where R⁵ is H and R⁶ is H or formyl, and optionally other substituents as herein defined. In Scheme 3, compounds of formula (VIII), in which the group R⁷R⁸N₃C is R⁴ when R⁴ is C₂₋₈ alkyl containing an azido substituent, may be prepared by reacting compounds of formula (VII) with an aside (for example sodium azide) in a suitable solvent such as DMF. Compounds of formula (IX) may be prepared by reduction of asides of formula (VIII), for example by catalytic hydrogenation with a catalyst such as platinum oxide. Compounds of formula (X) may be prepared by reacting an amine of formula (IX) with a mixture of formic acid and an anhydride, for example acetic anhydride. Compounds of formula (VII) may be prepared as described in EP-A-0381330.

Scheme 4 shows how compounds of formula (II) may be prepared when R⁴ is cyclobutyl, cyclopentyl or cyclohexyl containing a fluoro substituent and optionally other substituents as herein defined. In Scheme 4, compounds of formula (XII), in which the group R⁹R¹⁰FC is R⁴ when R⁴ is cyclobutyl, cyclopentyl or cyclohexyl containing a fluoro substituent, may be prepared by treatment of alcohols of formula (XI) with a fluorinating agent for example diethylaminosulphurtrifluoride (DAST). Compounds of formula (XI) may be prepared as described in Scheme 1.

Scheme 5 shows how compounds of formula (II) may be prepared when R⁴ is C₂₋₈ alkyl containing a nitro substituent and optionally other substituents as herein defined. In Scheme 5, compounds of formula (XIII), in which the group R⁷R⁸NO₂C is R⁴ when R⁴ is C₂₋₈ alkyl containing a nitro group, may be prepared by reacting compounds of formula (VII) with a metallic nitrite, for example sodium nitrite, in the presence of urea in a suitable solvent such as DMF.

Acid chlorides of formula (VI) may be prepared from carboxylic acids of formula (V) by reaction with a standard reagent such as oxalyl chloride in a suitable dry solvent such as THF or methylene chloride and with a catalytic quantity of DMF being added if necessary.

Carboxylic acids of formula (V), may be prepared from the appropriately substituted 4-aminobenzoic acid (IV) by reaction with an acid chloride R⁴COCl in water in the presence of at least two equivalents of a base such as an alkali metal carbonate or hydroxide (for example sodium bicarbonate). The substituted 4-aminobenzoic acids (IV) can generally be made by methods described in the literature.

The compounds of the invention may also be prepared using methods and techniques described in EP-A-0381330 and in UK Applications Nos. 9016580.4 and 9016581.2 and applications claiming priority therefrom.

In a further aspect, the invention provides processes as herein described for preparing the compounds of the invention.

The compounds of the invention show fungicidal activity across a range of plant diseases. They are, however, particularly active against the class of pathogens known as the phycomycetes (equivalent to the oomycetes). These include species of Phytophthora, Plasmopara, Peronospora and Pseudoperonospora. Examples of pathogens which the invention compounds are particularly useful for controlling are: Plasmopara viticola on vines; other downy mildews such as Bremia lactucae on lettuce; Peronospora spp. on soybeans, tobacco, onions and other hosts; Pseudoperonospora humuli on hops and Pseudoperonospora cubensis on cucurbits; Phytophthora infestans on potatoes and tomatoes and other Phytophthora spp. on vegetables, strawberries, avocado, pepper, ornamentals, tobacco, cocoa and other hosts; and Pythium sp on rice, horticultural plants, vegetables and turf.

The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems. The invention compounds may also be injected into plants or sprayed onto vegetation using electrodynamic spraying techniques or other low volume methods.

The term "plant" as used herein includes seedlings, bushes and trees. Furthermore, the fungicidal method of the invention includes preventative, protectant, prophylactic and eradicant treatments.

The compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrolidone, propylene glycol or N,N-dimethylformamide). The compositions may also be in the form of wet table powders or water dispersible granules comprising wetting or dispersing agents to facilitate the dispersion in liquids. The powders and granules may also contain fillers and suspending agents.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as cyclohexanone and methylcyclohexanone, chlorinated hydrocarbons such as chlorobenzene and trichlorethane, and alcohols such as benzyl alcohol, furfuryl alcohol, butanol and glycol ethers.

Suspension concentrates of largely insoluble solids may be prepared by ball or bead milling with a dispersing agent with a suspending agent included to stop the solid settling.

Compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives. For example, certain mineral oil and natural plant oil (such as soya bean and rape seed oil) additives have been found to enhance several-fold foliar protectant activity against, for example, Plasmopara viticola.

The invention compounds can be used as mixtures with fertilisers (e.g. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising a fertiliser and the compound of general formula (I) or a salt or metal complex thereof.

Wettable powders, emulsifiable concentrates and suspension concentrates will normally contain surfactants, e.g. a wetting agent, dispersing agent, emulsifying agent or suspending agent. These agents can be cationic, anionic or non-ionic agents.

Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example, polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient. After dilution to form aqueous preparations, such preparations may contain varying amounts of the active ingredient depending upon the intended purpose, but an aqueous preparation containing 0.0005%- or 0.01% to 10% by weight of active ingredient may be used.

The compositions of this invention may contain other compounds having biological activity, e.g. compounds having similar or complementary fungicidal activity or which possess plant growth regulating, herbicidal or insecticidal activity.

A fungicidal compound which may be present in the composition of the invention may be one which is capable of combating ear diseases of cereals (e.g. wheat) such as Septoria, Gibberella and Helminthosporium spp., seed and soil-borne diseases and downy and powdery mildews on grapes and powdery mildew and scab on apple, etc. By including another fungicide, the composition can have a broader spectrum of activity than the compound of general formula (I) alone. Further the other fungicide can have a synergistic effect on the fungicidal activity of the compound of general formula (I). Examples of fungicidal compounds which may be included in the composition of the invention are (RS)-1-aminopropylphosphonic acid, (RS)-4--(4-chlorophenyl)-2-phenyl-2-(1H-1,2,4-triazol-1-ylmethyl)butyronitrile, (Z)-N-but-2-enyloxymethyl-2-chloro-2',6'-diethylacetanilide, 1-(2-cyano-2--methoxyiminoacetyl)-3-ethyl urea, 3-(2,4-dichlorophenyl)-2-(1H-1,2,4-triazol-1-yl)quinazolin-4(3H)-one, 4-bromo-2-cyano-N,N-dimethyl-6-trifluoromethylbenzimidazole-1-sulphonamide, 5-ethyl-5,8-dihydro-8-oxo(1,3)-dioxol(4,5-g)quinoline-7-carboxylic acid, α-[N-(3-chloro-2,6-xylyl)-2-methoxyacetamido]-γ-butyrolactone, aldimorph, anilazine, benalaxyl, benomyl, biloxazol, binapacryl, bitertanol, blasticidin S, bromuconazole, bupirimate, buthiobate, captafol, captan, carbendazim, carboxin, chlorbenzthiazone, chloroneb, chlorothalonil, chlorozolinate, copper containing compounds such as copper oxychloride, copper sulphate and Bordeaux mixture, cycloheximide, cymoxanil, cyproconazole, cyprofuram, di-2-pyridyl disulphide 1,1'-dioxide, dichlofluanid, dichlone, diclobutrazol, diclomezine, dicloran, difenoconazole, dimethamorph, dimethirimol, diniconazole, dinocap, ditalimfos, dithianon, dodemorph, dodine, edifenphos, etaconazole, ethirimol, ethyl (Z)-N-benzyl-N-([methyl(methylthioethylideneamino-oxycarbonyl)amino]thio)-β-alaninate, etridiazole, fenapanil, fenarimol, fenfuram, fenpiclonil, fenpropidin, fenpropimorph, fentin acetate, fentin hydroxide, flutolanil, flutriafol, flusilazole, folpet, fosetyl-aluminium, fuberidazole, furalaxyl, furconazole-cis, guazatine, hexaconazole, hydroxyisoxazole, imazalil, imibenconazole, iprobenfos, iprodione, isoprothiolane, kasugamycin, mancozeb, maneb, mepanipyrim, mepronil, metalaxyl, methfuroxam, metsulfovax, myclobutanil, neoasozin, nickel dimethyldithiocarbamate, nitrothal-isopropyl, nuarimol, ofurace, organomercury compounds, oxadixyl, oxycarboxin, pefurazoate, penconazole, pencycuron, phenazin oxide, phthalide, polyoxin D, polyram, probenazole, prochloraz, procymidone, propamocarb, propiconazole, propineb, prothiocarb, pyrazophos, pyrifenox, pyroquilon, pyroxyfur, pyrrolnitrin, quinomethionate, quintozene, SSF-109, streptomycin, sulphur, tebuconazole, techlofthalam, tecnazene, tetraconazole, thiabendazole, thicyofen, thiophanate-methyl, thiram, tolclofos-methyl, triacetate salt of 1,1'-iminodi(octamethylene)diguanidine, triadimefon, triadimenol, triazbutyl, tricyclazole, tridemorph, triforine, validamycin A, vinclozolin, zarilamid and zineb. The compounds of general formula (I) can be mixed with soil, peat or other rooting media for the protection of plants against seed-borne, soil-borne or foliar fungal diseases.

Suitable insecticides which may be incorporated in the composition of the invention include buprofezin, carbaryl, carbofuran, carbosulfan, chlorpyrifos, cycloprothrin, demeton-s-methyl, diazinon, dimethoate, ethofenprox, fenitrothion, fenobucarb, fenthion, formothion, isoprocarb, isoxathion, monocrotophos, phenthoate, pirimicarb, propaphos and XMC.

Plant growth regulating compounds are compounds which control weeds or seedhead, formation, or selectively control the growth of less desirable plants (e.g. grasses).

Examples of suitable plant growth regulating compounds for use with the invention compounds are 3,6-dichloropicolinic acid, 1-(4-chlorophenyl)-4,6-di-methyl-2-oxo-1,2-dihydropyridine-3-carboxylic acid, methyl-3,6-dichloroanisate, abscisic acid, asulam, benzoylprop-ethyl, carbetamide, daminozide, difenzoquat, dikegulac, ethephon, fenpentezol, fluoridamid, glyphosate, glyphosine, hydroxybenzonitriles (e.g. bromoxynil), inabenfide, isopyrimol, long chain fatty alcohols and acids, maleic hydrazide, mefluidide, morphactins (e.g. chlorfluoroecol), paclobutrazol, phenoxyacetic acids (e.g. 2,4-D or MCPA), substituted benzoic acid (e.g. triiodobenzoic acid), substituted quaternary ammonium and phosphonium compounds (e.g. chloromequat, chlorphonium or mepiquatchloride), tecnazene, the auxins (e.g. indoleacetic acid, indolebutyric acid, naphthylacetic acid or naphthoxyacetic acid), the cytokinins (e.g. benzimidazole, benzyladenine, benzylaminopurine, diphenylurea or kinetin), the gibberellins (e.g. GA₃, GA₄ or GA₇) and triapenthenol.

The following Examples illustrate the invention.

Where shown, infrared and NMR data are selective; no attempt is made to list every absorption in all cases. The following abbreviations are used throughout:

| | |
|---|---|
| THF = tetrahydrofuran | |
| DMF = N,N-dimethylformamide | s = singlet |
| d = doublet | dd = doublet of doublets |
| m = multiplet | t = triplet |
| NMR = nuclear magnetic resonance | mpt = melting point |

### EXAMPLE 1

This Example illustrates the preparation of 2-chloro-4-(1-methylcyclobutanecarboxamido)-N,N dimethylbenzamide (compound No. 3 of Table 1).

4-amino-2-chloro-N,N-dimethylbenzamide (0.50g) was stirred in dry THF (15ml) at 0-5°C, with triethylamine (0.35g). To this mixture was added 1-methylcyclobutanecarbonyl chloride (0.34g), dropwise over 5 minutes, keeping the temperature below 10°C. The mixture was then warmed to room temperature and stood overnight. The reaction was poured into dilute hydrochloric acid, extracted with ethyl acetate, and the ethyl acetate layer washed with aqueous sodium bicarbonate, and then brine, before drying over magnesium sulphate, and evaporating to give a pale yellow solid (0.68g). This was recrystallised from ethyl acetate: chloroform, to give the desired product as a white crystalline solid, (0.448g), mp 202-203°C. NMR (CDCl₃, 270MHz) δ: 1.53(3H,s); 1.80-2.10(4H,m); 2.45-2.60(2H,m); 2.85(3H,s); 3.12(3H,s); 7.19(1H,d); 7.37(1H,dd); 7.70(1H,d) ppm.

### EXAMPLE 2

This Example illustrates the preparation of 2-chloro-4-(1-fluorocyclopentanecarboxamido)-N,N-dimethylbenzamide (compound No. 31 of Table I).

### Step 1

Preparation of 2-chloro-4-(1-hydroxycyclopentanecarboxamido)-N,N-dimethylbenzamide.

Dimethylaminopropyl ethyl carbodiimide hydrochloride (1.05g) was added to a stirred suspension of 4-amino-2-chloro-N,N-dimethylbenzamide (1.0g) and 1-hydroxycyclopentanecarboxylic acid (0.66g) in dichloromethane (10ml). After 1 hour, the solution was washed with 5% aqueous sodium bicarbonate, dried (MgSO₄) and concentrated under reduced pressure to give an off-white solid (500mg). Chromatography on silica, eluting with methanol in diethyl ether (2%), gave the desired product, mpt 199 - 200.5°C.

### Step 2

Preparation of 2-chloro-4-(1-fluorocyclopentanecarboxamido)-N,N-dimethylbenzamide.

2-Chloro-4-(1-hydroxycyclopentanecarboxamido)-N,N-dimethylbenzamide (180mg) was suspended in dichloromethane (2ml) at -50°C under nitrogen. To the stirred mixture was added diethylaminosulphurtrifluoride (DAST) (10»l) and the mixture was allowed to warm to room temperature. Further DAST was added until a clear solution was obtained (10»l required). After 30 minutes, the solution was diluted with dichloromethane and quenched with water. The separated organic phase was washed with 5% aqueous sodium bicarbonate, dried (MgSO₄) and concentrated under reduced presure to give the product as a colourless solid. Chromatography on silica, eluting with methanol in diethyl ether (2%), gave the title compound as a glass (40mg), ¹H NMR (270MHz; CDCl₃) δ 1.8-1.95(4H,m), 2.0-2.45(4H,m), 2.87(3H,s), 3.13(3H,s), 7.25(1H,d), 7.46(1H,dd), 7.84(1H,d), 8.3(1H,bd).

### EXAMPLE 3

This Example illustrates the preparation of 4-(2-azido-2-methylpropanamido)-2-chloro-N,N-dimethylbenzamide (compound No.22 of Table I).

4-(2-bromo-2-methylpropanamido)-2-chloro-N,N-dimethylbenzamide (1.0g) was dissolved in dry DMF (20ml), and sodium azide (206mg) was added. The mixture was heated at 90°C for 5 hours, then allowed to stand for 7 days before pouring into water (150ml). The aqueous layer was extracted with ethyl acetate, dried (MgSO₄) and concentrated under reduced pressure to give an orange solid (0.667g), which was purified by HPLC on silica. Elution with ethyl acetate gave the product as a white solid (489mg), mpt 127.5-128.5°C.

### EXAMPLE 4

This Example illustrates the preparation of 4-(2-amino-2-methylpropanamido)-2-chloro-N,N-dimethylbenzamide (compound No. 29 of Table I).

4-(2-azido-2-methylpropanamido)-2-chloro-N,N-dimethylbenzamide (0.80g) was hydrogenated in ethanol using platinum oxide as catalyst at 3 bar overpressure of hydrogen for 5.5 hours. The reaction mixture was filtered and concentrated under reduced presure. The residue was dissolved in dilute aqueous HCl, which was washed with chloroform and then made basic with dilute aqueous sodium hydroxide. The aqeous layer was extracted with chloroform, dried (MgSO₄) and concentrated under reduced pressure to give a gum (0.624g), which crystallised on standing. HPLC on silica, eluting with methanol, gave the desired product as a solid (154mg), mpt 135.5-137.5°C.

### EXAMPLE 5

This Example illustrates the preparation of 4-(2-formamido-2-methylpropanamido)-2-chloro-N,N-dimethylbenzamide (compound No. 30 of Table I).

Formic acid (210mg) was added dropwise to acetic anhydride (440mg) and stirred for 1.5 hours. The mixture was added to 4-(2-amino-2-methylpropanamido)-2-chloro-N,N-dimethylbenzamide (570mg) and the whole stirred until a solution resulted (ca 5 hours). The solution was concentrated under reduced pressure to give a gum which was triturated with diethyl ether. The product was obtained as a cream solid (410mg), mpt 139°C.

### EXAMPLE 6

This Example illustrates the preparation of 4-(2-nitro-2-methylpropanamido)-2-chloro-N,N-dimethylbenzamide (compound No. 23 of Table I).

A mixture of 4-(2-bromo-2-methylpropanamido)-2-chloro-N,N-dimethylbenzamide (0.87g), sodium nitrite (0.345g), urea (0.333g) in DMF (15ml) was heated at 60°C for 6 hours. The reaction mixture was quenched by pouring it into water, and was extracted with ethyl acetate. The organic layer was washed with brine, dried (MgSO₄) and concentrated under reduced pressure to give a yellow gum (0.834g). HPLC on silica, eluting with ethyl acetate, gave the product as a solid (270mg), Mpt 184-186°C.

The following are examples of compositions suitable for agricultural and horticultural purposes which can be formulated from the compounds of the invention. Such compositions form another aspect of the invention. Percentages are by weight.

### EXAMPLE 7

An emulsifiable concentrate is made up by mixing and stirring the ingredients until all are dissolved.

| | |
|---|---|
| Compound No. 3 of Table I | 10% |
| Benzyl alcohol | 30% |
| Calcium dodecylbenzenesulphonate | 5% |
| Nonylphenolethoxylate (13 mole ethylene oxide) | 10% |
| Alkyl benzenes | 45% |

### EXAMPLE 8

The active ingredient is dissolved in methylene dichloride and the resultant liquid sprayed on to the granules of attapulgite clay. The solvent is then allowed to evaporate to produce a granular composition.

| | |
|---|---|
| Compound No. 3 of Table I | 5% |
| Attapulgite granules | 95% |

### EXAMPLE 9

A composition suitable for use as a seed dressing is prepared by grinding and mixing the three ingredients.

| | |
|---|---|
| Compound No. 3 of Table I | 50% |
| Mineral oil | 2% |
| China clay | 48% |

### EXAMPLE 10

A dustable powder is prepared by grinding and mixing the active ingredient with talc.

| | |
|---|---|
| Compound No. 3 of Table I | 5% |
| Talc | 95% |

### EXAMPLE 11

A suspension concentrate is prepared by ball milling the ingredients to form an aqueous suspension of the ground mixture with water.

| | |
|---|---|
| Compound No. 3 of Table I | 40% |
| Sodium lignosulphonate | 10% |
| Bentonite clay | 1% |
| Water | 49% |

This formulation can be used as a spray by diluting into water or applied directly to seed.

### EXAMPLE 12

A wettable powder formulation is made by mixing together and grinding the ingredients until all are thoroughly mixed.

| | |
|---|---|
| Compound No. 3 of Table I | 25% |
| Sodium lauryl sulphate | 2% |
| Sodium lignosulphonate | 5% |
| Silica | 25% |
| China clay | 43% |

### EXAMPLE 13

The compounds were tested against a variety of foliar fungal diseases of plants. The technique employed was as follows.

The plants were grown in John Innes Potting Compost (No. 1 or 2) in 4cm diameter minipots. The test compounds were formulated either by bead milling with aqueous Dispersol T or as a solution in acetone or acetone/ethanol which was diluted to the required concentration immediately before use. For the foliage diseases, the formulations (100 ppm active ingredient) were sprayed onto the foliage and applied to the roots of the plants in the soil. The sprays were applied to maximum retention and the root drenches to a final concentration equivalent to approximately 40 ppm a.i. in dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading:
4 = no disease
3 = trace-5% of disease on untreated plants
2 = 6-25% of disease on untreated plants
1 = 26-59% of disease on untreated plants
0 = 60-100% of disease untreated plants
The results are shown in Table II.

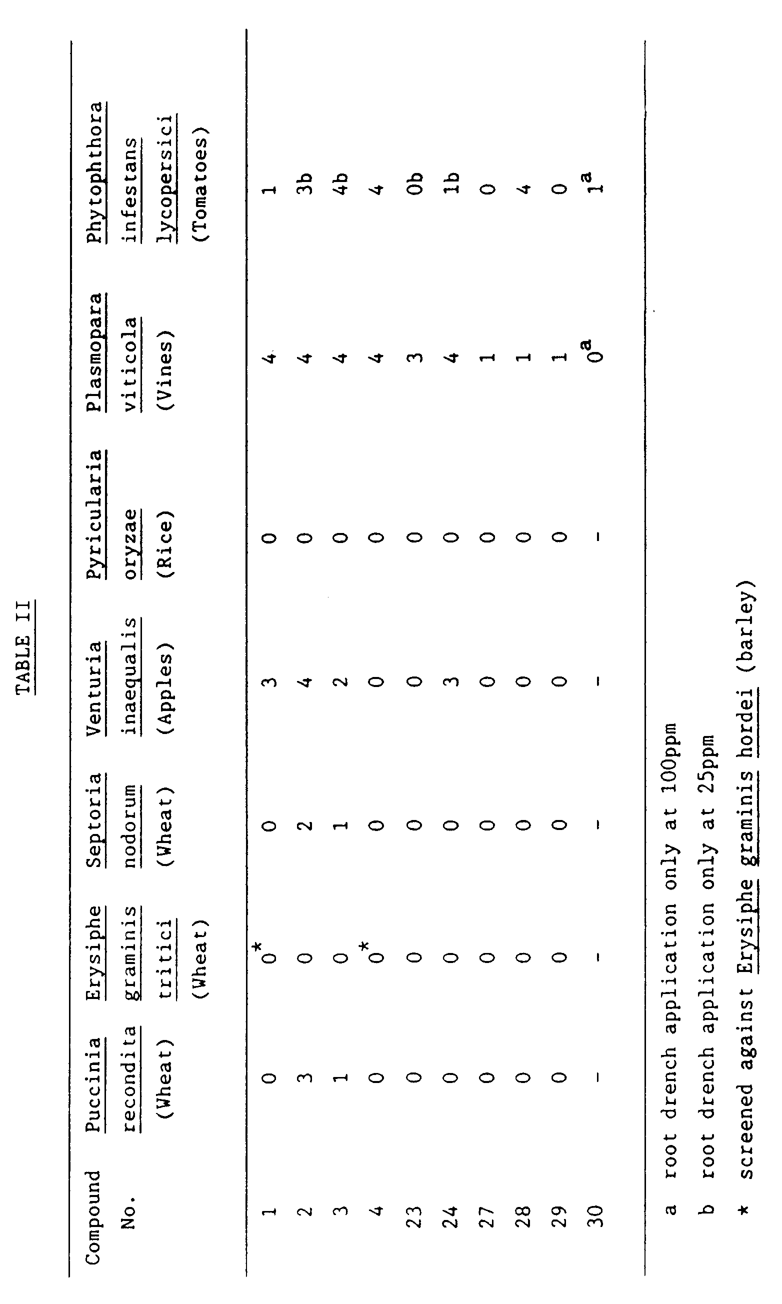

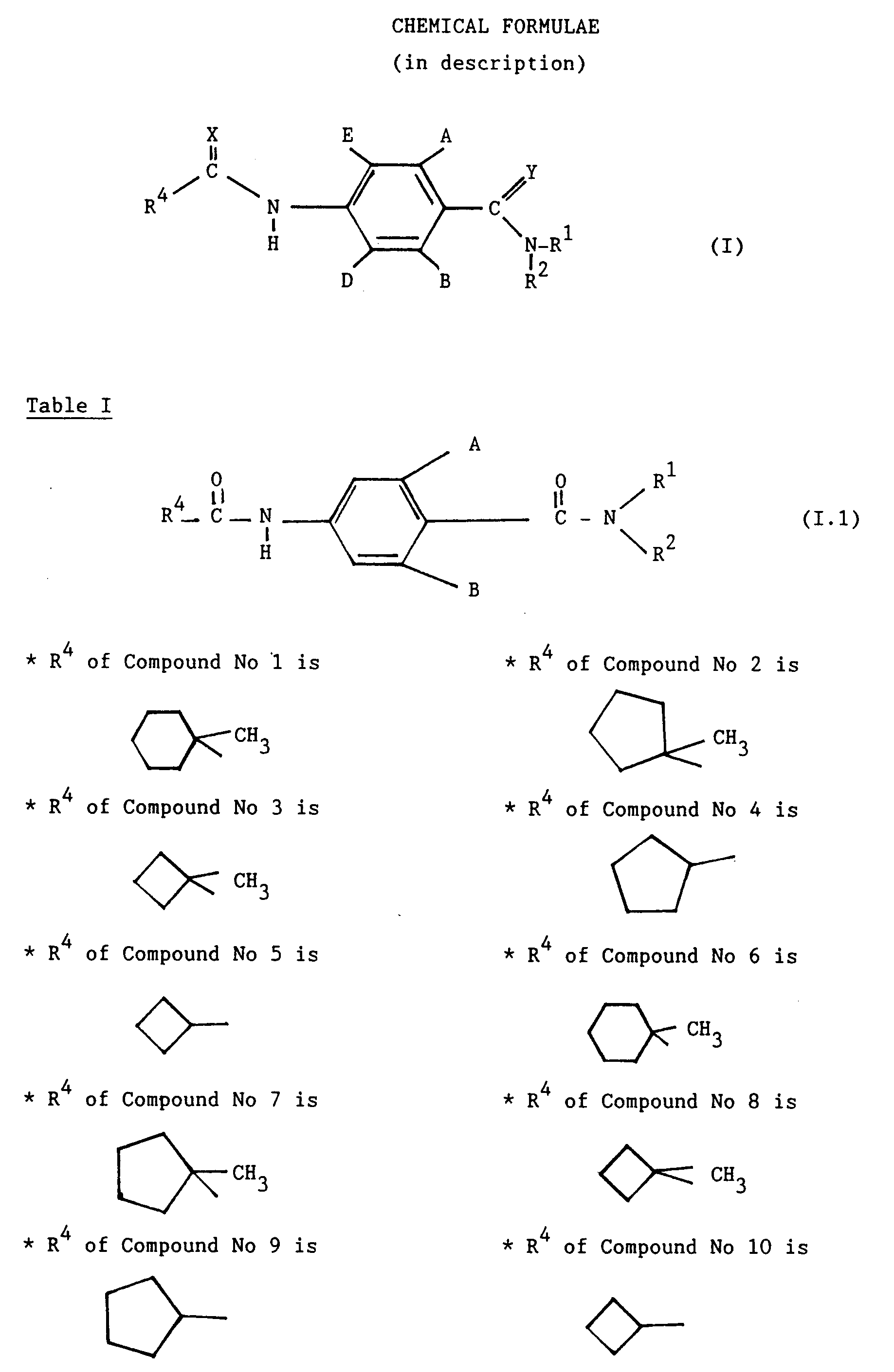

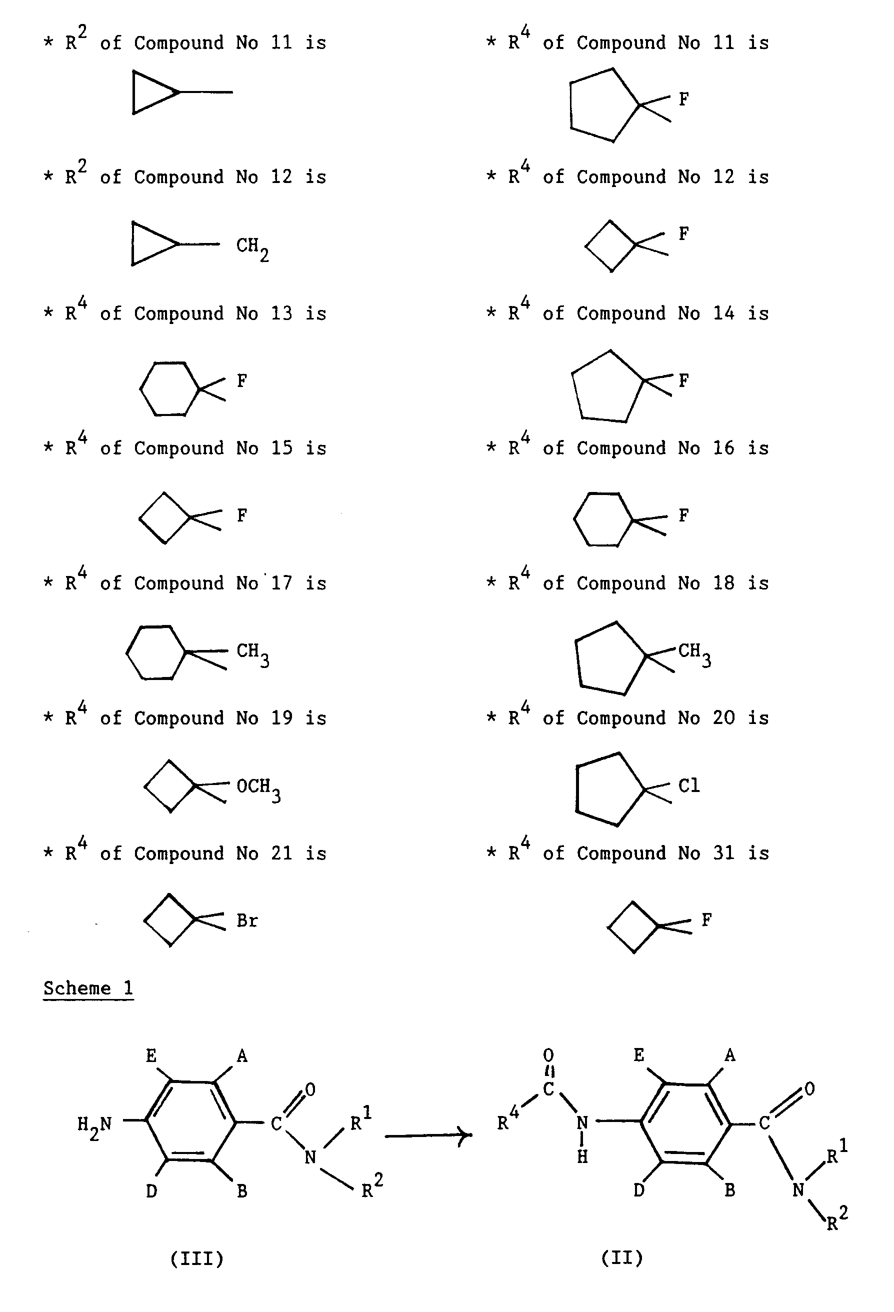

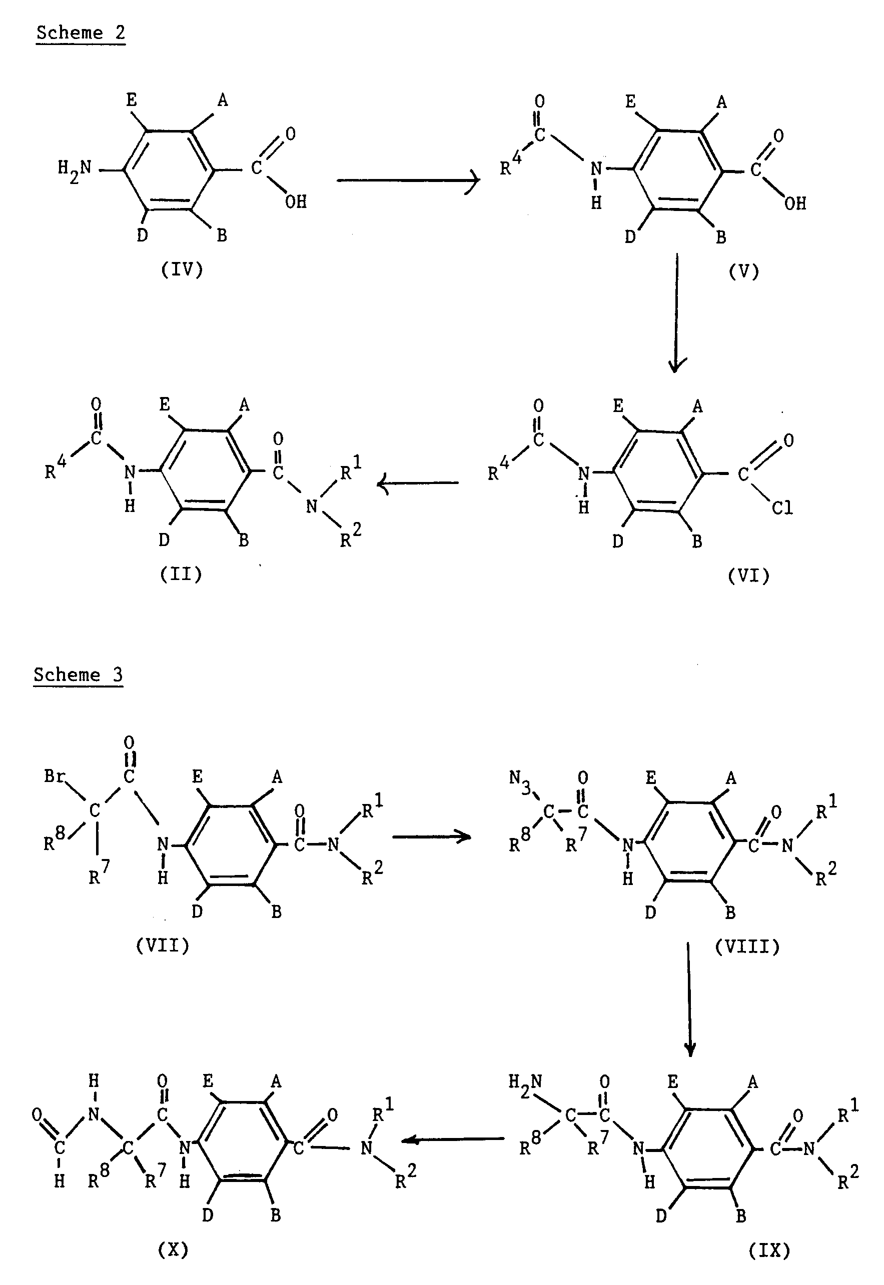

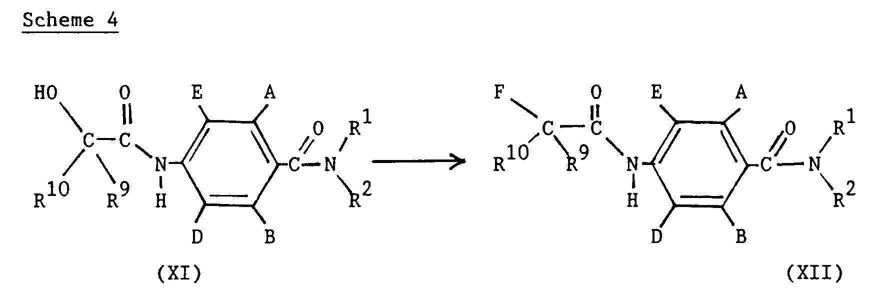

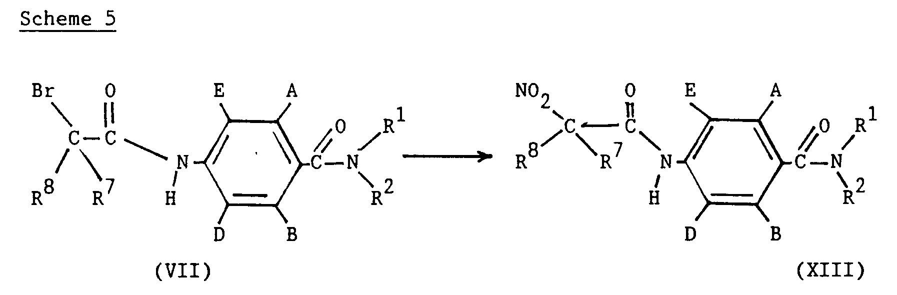

## Claims

1. A compound of the formula (I); 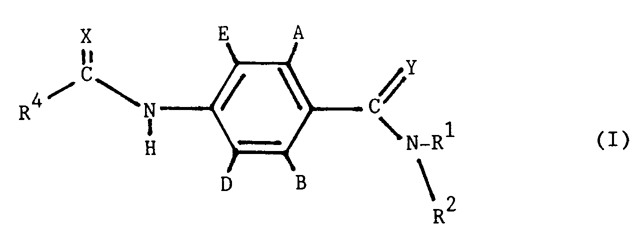 in which A and B are independently H, fluoro, chloro, bromo, iodo, C₁₋₄ alkyl, C₁₋₄ alkoxy, halo(C₁₋₄)alkyl, cyano, C₁₋₄ alkoxycarbonyl, C₁₋₄ alkoxymethyl, C₁₋₄ alkylthiomethyl, C₂₋₄ alkenyl, C₂₋₄ alkynyl, formyl, C₁₋₄ alkylthio, carboxy, C₁₋₄ alkylcarbonyl or nitro, provided that both A and B are not H; D and E are independently H or fluoro; R¹ and R² are independently C₁₋₄ alkyl, C₃₋₆ alkenyl (optionally substituted with halogen), C₃₋₆ alkynyl (optionally substituted with halogen), C₁₋₄ alkoxy, C₁₋₄ haloalkyl, C₃₋₆ cycloalkyl, C₃₋₆ cycloalkyl(C₁₋₄)alkyl, C₁₋₄ alkoxy(C₁₋₄)alkyl, C₁₋₄ alkylthio(C₁₋₄₎-alkyl, or cyano; or R¹ and R² together with the nitrogen atom to which they are attached join to form a morpholine, piperidine, pyrrolidine or azetidine ring which is optionally substituted with C₁₋₄ alkyl; R⁴ is cyclobutyl, cyclopentyl or cyclohexyl, any of which is optionally substituted with halogen, C₁₋₄ alkyl, C₁₋₄ alkoxy, C₁₋₄ alkylthio, C₂₋₄ alkenyl, C₂₋₄ alkynyl, cyano, C₁₋₄ alkylcarbonyl, C₁₋₄ alkoxycarbonyl, azido, nitro, isocyano or NR⁵R⁶ (where R⁵ and R⁶ are independently H, C₁₋₄ alkyl, C₁₋₄ alkylcarbonyl or formyl) or R⁴ is C₂₋₈ alkyl substituted with azido, nitro, isocyano, -NR⁵R⁶ (where R⁵ and R⁶ are independently C₁₋₄ alkyl, C₁₋₄ alkylcarbonyl or formyl), cyano, C₁₋₄ alkylcarbonyl or C₁₋₄ alkoxycarbonyl; and X and Y are independently oxygen or sulphur.

2. A compound of the formula (I): 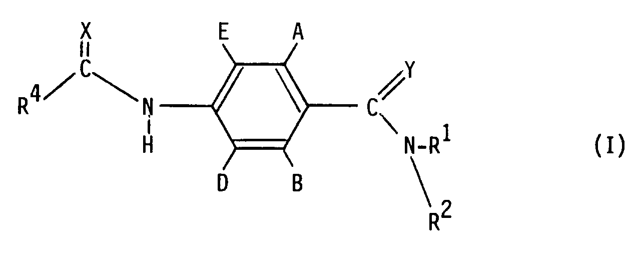 in which A is fluoro, chloro, bromo, iodo, C₁₋₄ alkyl, halo(C₁₋₄)alkyl, C₁₋₄ alkoxy, C₁₋₄ alkylthio, cyano, nitro, C₁₋₄ alkoxycarbonyl, C₁₋₄ alkoxy(C₁₋₄)alkyl, C₁₋₄ alkylthio(C₁₋₄)alkyl, C₂₋₄ alkenyl, C₂₋₄ alkynyl or C₁₋₄ alkylcarbonyl; B is H; D and E are independently H or fluoro; R¹ is methyl; R² is C₁₋₄ alkyl, halo(C₁₋₄)alkyl, C₃₋₄ alkenyl, C₃₋₄ alkynyl, cyclopropyl, cyclopropyl(C₁₋₄)alkyl, C₁₋₄ alkoxy, C₁₋₄ alkoxy(C₁₋₄)alkyl, C₁₋₄ alkylthio(C₁₋₄)alkyl or cyano; R⁴ is cyclobutyl, cyclopentyl or cyclohexyl (each optionally substituted in the 1-position with halogen, C₁₋₄ alkyl or C₁₋₄ alkoxy), or the group R³(CH₃)₂C- in which R³ is azido, nitro, cyano, NR⁵R⁶ (where R⁵ and R⁶ are independently H, C₁₋₄ alkyl or formyl), C₁₋₄ alkoxycarbonyl or C₁₋₄ alkylcarbonyl; and X and Y are both O.

3. A compound of formula (I): 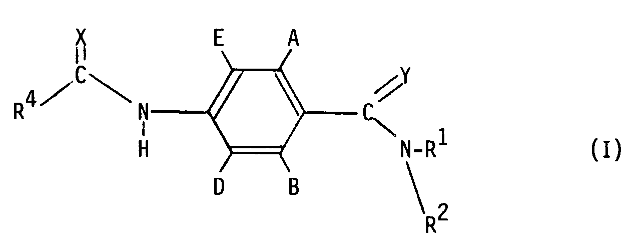 in which A is fluoro, chloro, bromo, iodo or methyl; B is H, fluoro or chloro; D and E are both H; R¹ and R² are both methyl; R⁴ is cyclobutyl, cyclopentyl or cyclohexyl (each optionally substituted in the 1-position with halogen, C₁₋₄ alkyl or C₁₋₄ alkoxy), or the group R³(CH₃)₂C- in which R³ is azido, nitro, cyano, NR⁵R⁶ (where R⁵ and R⁶ are independently H, C₁₋₄ alkyl or formyl), C₁₋₄ alkoxycarbonyl or C₁₋₄ alkylcarbonyl; and X and Y are both oxygen.

4. A compound according to claim 1 in which A is fluoro, chloro, bromo, iodo or methyl; B is H, fluoro or chloro; D and E are both H; R¹ and R² are both methyl; R⁴ is 1-methylcyclobutyl, 1-methylcyclopentyl or 1-methylcyclohexyl; and X and Y are both oxygen.

5. A process for preparing a compound according to claim 1, 2 or 3 in which X and Y are both oxygen, which comprises reacting in a suitable organic solvent in the presence of a base either:
(a) a compound of formula (III): 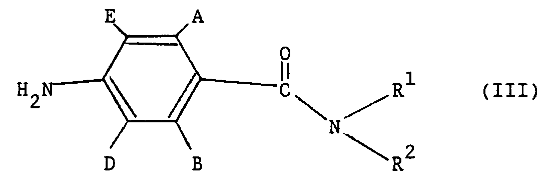 with an acid chloride R⁴COCl; or
(b) a compound of formula (VI): 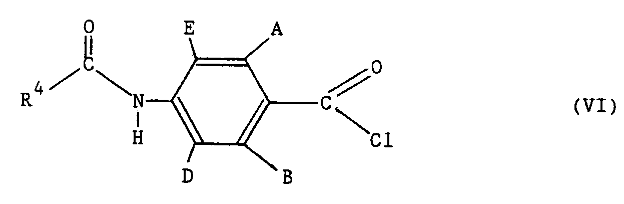 with an amine R¹R²NH; wherein A, B, D, E, R¹ R² and R⁴ have the meanings given in claim 1, 2 or 3.

6. A process for preparing a compound according to claim 1, 2 or 3 in which X and Y are both oxygen, which comprises:
(a) when R⁴ is C₂₋₈ alkyl containing an azido substituent,
reacting a compound of formula (VII): 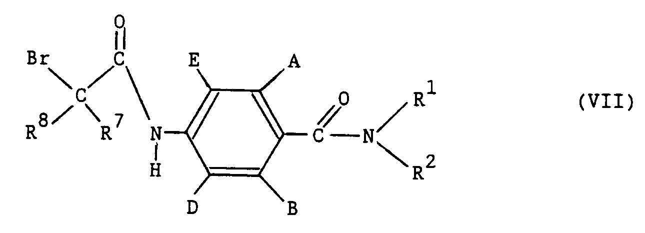 with an azide in a suitable solvent, or
(b) when R⁴ is C₂₋₈ alkyl containing a substituent -NR⁵R⁶ where R⁵ and R⁶ are both H,
reducing an azide of formula (VIII): 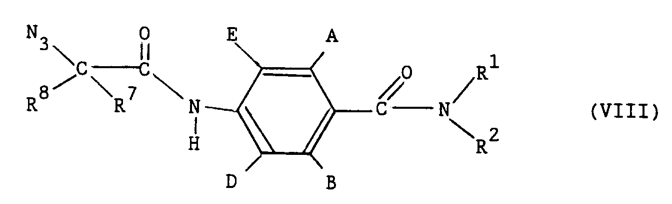
(c) when R⁴ is C₂₋₈ alkyl containing a substituent -NR⁵R⁶ where R⁵ is H and R⁶ is formyl,
reacting an amine of formula (IX): 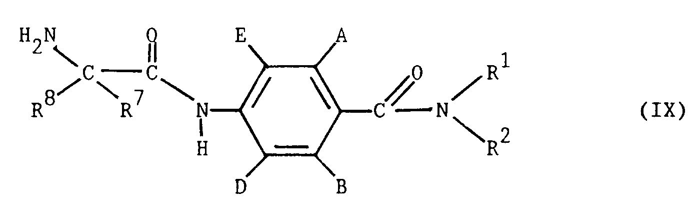 with a mixture of formic acid and an anhydride, or
(d) when R⁴ is cyclobutyl, cyclopentyl or cyclohexyl containing a fluoro substituent,
treating an alcohol of formula (XI): 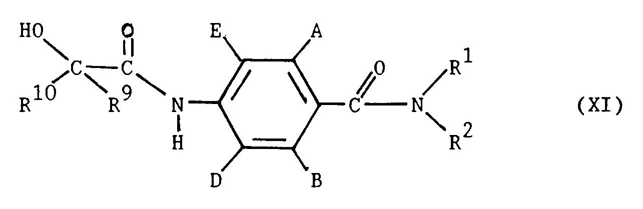 with a fluorinating agent, or
(e) when R⁴ is C₂₋₈ alkyl containing a nitro subsititent,
reacting a compound of formula (VII): 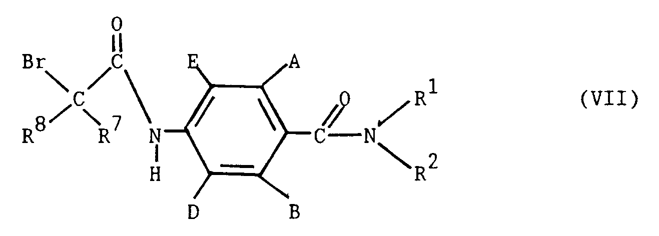 with a metallic nitrite in the presence of urea in a suitable solvent; wherein A, B, D, E, R¹, R² and R⁴ have the meanings given in claim 1, the group R⁷R⁸C is the residue of R⁴ when R⁴ is C₂₋₈ alkyl containing a bromo group in the compound of formula (VII), containing an azido group in the compound of formula (VIII), and containing an amino group in the compound of formula (IX), and R⁹R¹⁰C is the residue of R⁴ when R⁴ is cyclobutyl, cyclopentyl or cyclohexyl containing a hydroxy group in the compound of formula (XI).

7. A fungicidal composition comprising a fungicidally effective amount of a compound according to claim 1, 2 or 3 and a fungicidally acceptable carrier or diluent therefor.

8. A method of combating fungi which comprises applying to plants, to the seeds of plants or to the locus of the plants or seeds, a compound according to claim 1, 2 or 3 or a composition according to claim 7.

## Patentansprüche

1. Verbindung mit der folgenden Formel (I): 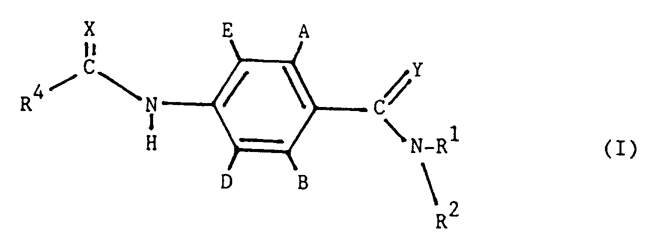 in der A und B unabhängig voneinander für H, Fluor, Chlor, Brom, Jod, C₁₋₄-Alkyl, C₁₋₄-Alkoxy, Halogen(C₁₋₄)-alkyl, Cyano, C₁₋₄-Alkoxycarbonyl, C₁₋₄-Alkoxymethyl, C₁₋₄-Alkylthiomethyl, C₂₋₄-Alkenyl, C₂₋₄-Alkinyl, Formyl, C₁₋₄-Alkylthio, Carboxy, C₁₋₄-Alkylcarbonyl oder Nitro stehen, mit der Maßgabe, daß nicht sowohl A als auch B für H steht; D und E unabhängig voneinander für H oder Fluor stehen; R¹ und R² unabhängig voneinander für C₁₋₄-Alkyl, C₃₋₆-Alkenyl (gegebenenfalls mit Halogen substituiert), C₃₋₆-Alkinyl (gegebenenfalls mit Halogen substituiert), C₁₋₄-Alkoxy, C₁₋₄-Halogenalkyl, C₃₋₆-Cycloalkyl, C₃₋₆-Cycloalkyl(C₁₋₄)alkyl, C₁₋₄-Alkoxy(C₁₋₄)alkyl, C₁₋₄-Alkylthio(C₁₋₄)alkyl oder Cyano stehen; oder in der R¹ und R² zusammen mit dem Stickstoffatom, an das sie gebunden sind, unter Bildung eines Morpholin-, Piperidin-, Pyrrolidin- oder Asetidin-Rings, der gegebenenfalls mit C₁₋₄-Alkyl substituiert ist, miteinander verbunden sind; R⁴ für Cyclobutyl, Cyclopentyl oder Cyclohexyl steht, die jeweils gegebenenfalls mit Halogen, C₁₋₄-Alkyl, C₁₋₄-Alkoxy, C₁₋₄ Alkylthio, C₂₋₄-Alkenyl, C₂₋₄-Alkinyl, Cyano, C₁₋₄-Alkylcarbonyl, C₁₋₄-Alkoxycarbonyl, Azido, Nitro, Isocyano oder NR⁵R⁶ (wobei R⁵ und R⁶ unabhängig voneinander für H, C₁₋₄-Alkyl, C₁₋₄-Alkylcarbonyl oder Formyl stehen) substituiert sind, oder in der R⁴ für C₂₋₈-Alkyl steht, das mit Azido, Nitro, Isocyano, -NR⁵R⁶ (wobei R⁵ und R⁶ unabhängig voneinander für C₁₋₄-Alkyl, C₁₋₄-Alkylcarbonyl oder Formyl stehen), Cyano, C₁₋₄-Alkylcarbonyl oder C₁₋₄-Alkoxycarbonyl substituiert ist; und in der X und Y unabhängig voneinander für Sauerstoff oder Schwefel stehen.

2. Verbindung mit der folgenden Formel (I): 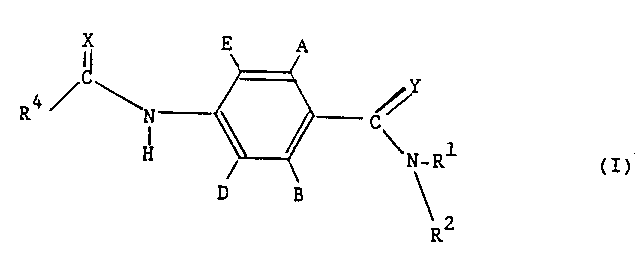 in der A für Fluor, Chlor, Brom, Jod, C₁₋₄-Alkyl, Halogen(C₁₋₄)alkyl, C₁₋₄-Alkoxy, C₁₋₄-Alkylthio, Cyano, Nitro, C₁₋₄-Alkoxycarbonyl, C₁₋₄-Alkoxy(C₁₋₄)-alkyl, C₁₋₄-Alkylthio(C₁₋₄)alkyl, C₂₋₄-Alkenyl, C₂₋₄-Alkinyl oder C₁₋₄-Alkylcarbonyl steht; B für H steht; D und E unabhängig voneinander für H oder Fluor stehen; R¹ für Methyl steht; R² für C₁₋₄-Alkyl, Halogen(C₁₋₄)alkyl, C₃₋₄-Alkenyl, C₃₋₄-Alkinyl, Cyclopropyl, Cyclopropyl(C₁₋₄)alkyl, C₁₋₄-Alkoxy, C₁₋₄-Alkoxy(C₁₋₄)alkyl, C₁₋₄-Alkylthio(C₁₋₄)alkyl oder Cyano steht; R⁴ für Cyclobutyl, Cyclopentyl oder Cyclohexyl (die jeweils gegebenenfalls in der 1-Stellung mit Halogen, C₁₋₄-Alkyl oder C₁₋₄-Alkoxy substituiert sind) oder für die Gruppe R³(CH₃)₂C- steht, in der R³ für Azido, Nitro, Cyano, NR⁵R⁶ (wobei R⁵ und R⁶ unabhängig voneinander für H, C₁₋₄-Alkyl oder Formyl stehen), C₁₋₄-Alkoxycarbonyl oder C₁₋₄-Alkylcarbonyl steht; und in der X und Y beide für O stehen.

3. Verbindung mit der folgenden Formel (I): 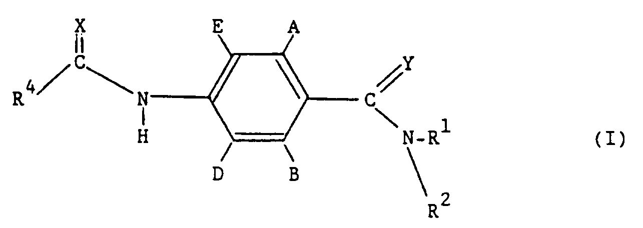 in der A für Fluor, Chlor, Brom, Jod oder Methyl steht; B für H, Fluor oder Chlor steht; D und E beide für H stehen; R¹ und R² beide für Methyl stehen; R⁴ für Cyclobutyl, Cyclopentyl oder Cyclohexyl (die jeweils gegebenenfalls in der 1-Stellung mit Halogen, C₁₋₄-Alkyl oder C₁₋₄-Alkoxy substituiert sind) oder für die Gruppe R³(CH₃)₂C- steht, in der R³ für Azido, Nitro, Cyano, NR⁵R⁶ (wobei R⁵ und R⁶ unabhängig voneinander für H, C₁₋₄-Alkyl oder Formyl stehen), C₁₋₄-Alkoxycarbonyl oder C₁₋₄-Alkylcarbonyl steht; und in der X und Y beide für Sauerstoff stehen.

4. Verbindung nach Anspruch 1, wobei A für Fluor, Chlor, Brom, Jod oder Methyl steht; B für H, Fluor oder Chlor steht; D und E beide für H stehen; R¹ und R² beide für Methyl stehen; R⁴ für 1-Methylcyclobutyl, 1-Methylcyclopentyl oder 1-Methylcyclohexyl steht; und X und Y beide für Sauerstoff stehen.

5. Verfahren zur Herstellung einer Verbindung nach Anspruch 1, 2 oder 3, wobei X und Y beide für Sauerstoff stehen, bei dem in einem geeigneten organischen Lösungsmittel in Gegenwart einer Base:
(a) entweder eine Verbindung mit der folgenden Formel (III): 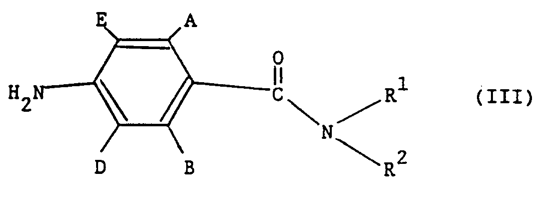 mit einem Säurechlorid R⁴COCl umgesetzt wird; oder
(b) eine Verbindung mit der folgenden Formel (VI): 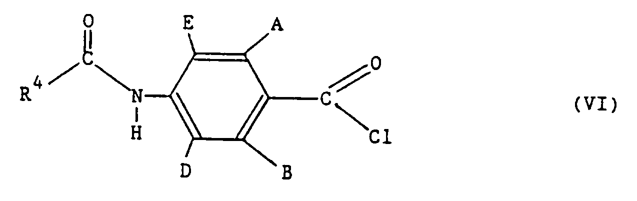 mit einem Amin R¹R²NH umgesetzt wird, wobei A, B, D, E, R¹, R² und R⁴ die in Anspruch 1, 2 oder 3 angegebenen Bedeutungen haben.

6. Verfahren zur Herstellung einer Verbindung nach Anspruch 1, 2 oder 3, wobei X und Y beide für Sauerstoff stehen, bei dem:
(a) wenn R⁴ für C₂₋₈-Alkyl steht, das einen Azido-Substituenten enthält, eine Verbindung mit der folgenden Formel (VII): 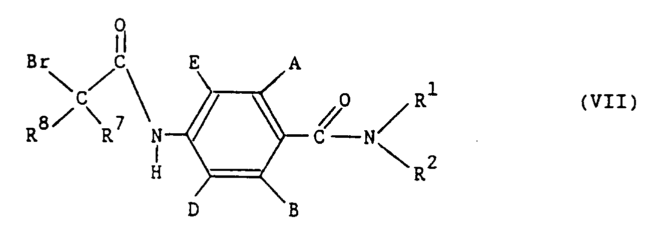 mit einem Azid in einem geeigneten Lösungsmittel umgesetzt wird, oder
(b) wenn R⁴ für C₂₋₈-Alkyl steht, das einen Substituenten -NR⁵R⁶ enthält, wobei R⁵ und R⁶ beide für H stehen, ein Azid mit der folgenden Formel (VIII) reduziert wird: 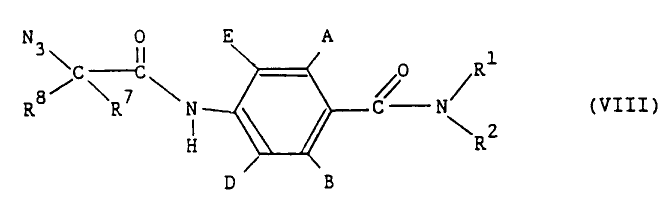
(c) wenn R⁴ für C₂₋₈-Alkyl steht, das einen Substituenten -NR⁵R⁶ enthält, wobei R⁵ für H und R⁶ für Formyl steht, ein Amin mit der folgenden Formel (IX): 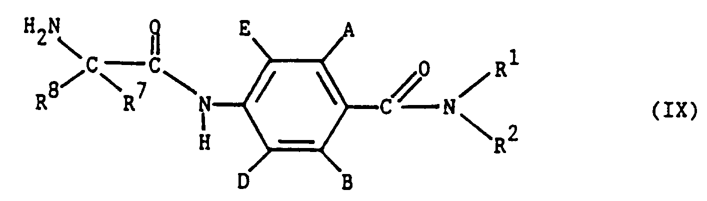 mit einem Gemisch aus Ameisensäure und einem Anhydrid umgesetzt wird, oder
(d) wenn R⁴ für Cyclobutyl, Cyclopentyl oder Cyclohexyl steht, das einen Fluor-Substituenten enthält, ein Alkohol mit der folgenden Formel (XI) : 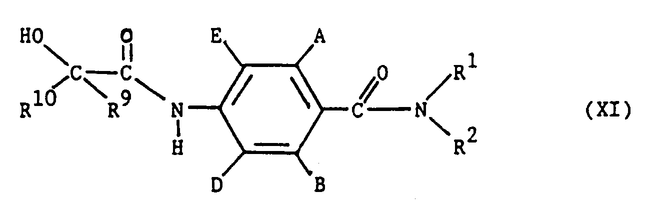 mit einem Fluorierungsmittel behandelt wird, oder
(e) wenn R⁴ für C₂₋₈-Alkyl steht, das einen Nitro-Substituenten enthält, eine Verbindung mit der folgenden Formel (VII): 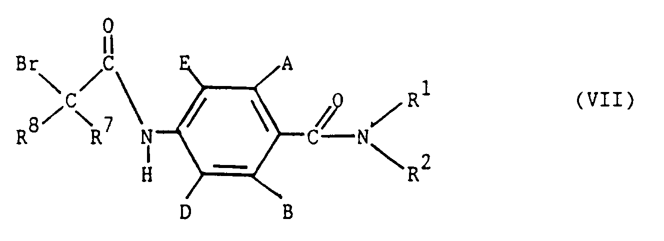 mit einem Metallnitrit in Gegenwart von Harnstoff in einem geeigneten Lösungsmittel umgesetzt wird, wobei A, B, D, E, R¹, R² und R⁴ die in Anspruch 1 angegebenen Bedeutungen haben, die Gruppe R⁷R⁸C für den Rest von R⁴ steht, wenn R⁴ für C₂₋₈-Alkyl steht, das in der Verbindung mit der Formel (VII) eine Brom-Gruppe enthält, in der Verbindung mit der Formel (VIII) eine Azido-Gruppe enthält und in der Verbindung mit der Formel (IX) eine Amino-Gruppe enthält, und R⁹R¹⁰C ist der Rest von R⁴, wenn R⁴ in der Verbindung mit der Formel (XI) für Cyclobutyl, Cyclopentyl oder Cyclohexyl steht, das eine Hydroxy-Gruppe enthält.

7. Fungicide Zusammensetzung, die eine fungicid wirksame Menge einer Verbindung nach Anspruch 1, 2 oder 3 und ein für Fungicide geeignetes Trägermittel oder Streckmittel dafür enthält.

8. Verfahren zur Pilzbekämpfung, bei dem auf Pflanzen, auf das Saatgut von Pflanzen oder auf den Standort der Pflanzen oder des Saatguts eine Verbindung nach Anspruch 1, 2 oder 3 oder eine Zusammensetzung nach Anspruch 7 aufgebracht wird.

## Revendications

1. Composé de formule (I) : 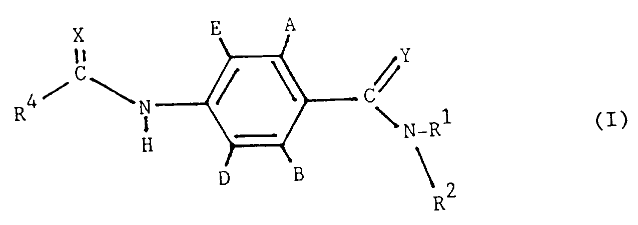 dans laquelle A et B représentent indépendamment H, un groupe fluoro, chloro, bromo, iodo, alkyle en C₁ à C₄, alkoxy en C₁ à C₄, halogénalkyle en C₁ à C₄, cyano, (alkoxy en C₁ à C₄)-carbonyle, (alkoxy en C₁ à C₄)-méthyle, (alkyle en C₁ à C₄)-thiométhyle, alcényle en C₂ à C₄, alcynyle en C₂ à C₄, formyle, alkylthio en C₁ à C₄, carboxy, (alkyle en C₁ à C₄)-carbonyle ou nitro, sous réserve que A et B ne représentent pas tous deux H ; D et E représentent indépendamment H ou un groupe fluoro, R¹ et R² représentent indépendamment un groupe alkyle en C₁ à C₄, alcényle en C₃ à C₆ (facultativement substitué avec un halogène), alcynyle en C₃ à C₆ (facultativement substitué avec un halogène), alkoxy en C₁ à C₄, halogénalkyle en C₁ à C₄, cycloalkyle en C₃ à C₆, (cycloalkyle en C₃ à C₆)-(alkyle en C₁ à C₄), (alkoxy en C₁ à C₄)-(alkyle en C₁ à C₄), (alkyle en C₁ à C₄) -thio- (alkyle en C₁ à C₄) ou cyano ; ou bien R¹ et R², conjointement avec l'atome d'azote auquel ils sont fixés, sont réunis en formant un noyau morpholine, pipéridine, pyrrolidine ou azétidine qui est facultativement substitué avec un groupe alkyle en C₁ à C₄ ; R⁴ représente un groupe cyclobutyle, cyclopentyle ou cyclohexyle, l'un quelconque de ces groupes étant facultativement substitué avec un halogène, un groupe alkyle en C₁ à C₄, alkoxy en C₁ à C₄, alkylthio en C₁ à C₄, alcényle en C₂ à C₄, alcynyle en C₂ à C₄, cyano, (alkyle en C₁ à C₄)-carbonyle, (alkoxy en C₁ à C₄)-carbonyle, azido, nitro, isocyano ou -NR⁵R⁶ (dans lequel R⁵ et R⁶ représentent indépendamment H, un groupe alkyle en C₁ à C₄, (alkyle en C₁ à C₄) -carbonyle ou formyle), ou bien R⁴ représente un groupe alkyle en C₂ à C₈ substitué avec un groupe azido, nitro, isocyano, NR⁵R⁶ (dans lequel R⁵ et R⁶ représentent indépendamment un groupe alkyle en C₁ à C₄, (alkyle en C₁ à C₄)-carbonyle ou formyle), cyano, (alkyle en C₁ à C₄)-carbonyle ou (alkoxy en C₁ à C₄)-carbonyle ; et X et Y représentent indépendamment l'oxygène ou le soufre.

2. Compose de formule (I) : 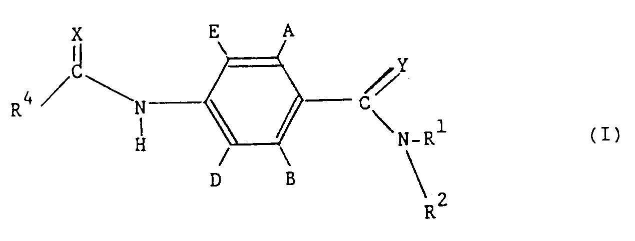 dans laquelle A représente un groupe fluoro, chloro, bromo, iodo, alkyle en C₁ à C₄, halogénalkyle en C₁ à C₄, alkoxy en C₁ à C₄, alkylthio en C₁ à C₄, cyano, nitro, (alkoxy en C₁ à C₄)-carbonyle, (alkoxy en C₁ à C₄)-(alkyle en C₁ à C₄), (alkyle en C₁ à C₄)-thio-(alkyle en C₁ à C₄), alcényle en C₂ à C₄, alcynyle en C₂ à C₄ ou (alkyle en C₁ à C₄)-carbonyle ; B représente H : D et E représentent indépendamment H ou un groupe fluoro ; R¹ représente un groupe méthyle ; R² représente un groupe alkyle en C₁ à C₄, halogénalkyle en C₁ à C₄, alcényle en C₃ ou C₄, alcynyle en C₃ ou C₄ cyclopropyle, cyclopropyl-(alkyle en C₁ à C₄), alkoxy en C₁ à C₄, (alkoxy en C₁ à C₄)-(alkyle en C₁ à C₄), (alkyle en C₁ à C₄)-thio-(alkyle en C₁ à C₄) ou cyano ; R⁴ représente un groupe cyclobutyle, cyclopentyle ou cyclohexyle (chacun facultativement substitué en position 1 avec un halogène, un groupe alkyle en C₁ à C₄ ou alkoxy en C₁ à C₄), ou le groupe R³(CH₃)₂C- dans lequel R³ représente un groupe azido, nitro, cyano, NR⁵R⁶ (dans lequel R⁵ et R⁶ représentent indépendamment H, un groupe alkyle en C₁ à C₄ ou formyle), (alkoxy en C₁ à C₄)-carbonyle ou (alkyle en C₁ à C₄)-carbonyle ; et X et Y représentent l'un et l'autre O.

3. Composé de formule (I) : 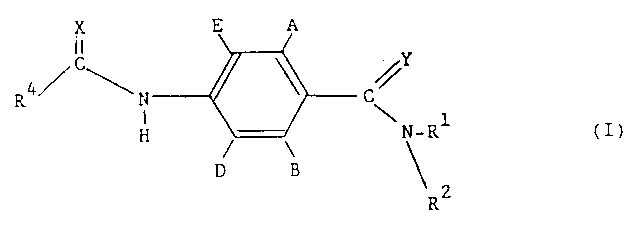 dans laquelle A représente un groupe fluoro, chloro, bromo, iodo ou méthyle ; B représente H, un groupe fluoro ou chloro, D et E représentent l'un et l'autre H ; R¹ et R² représentent l'un et l'autre un groupe méthyle ; R⁴ représente un groupe cyclobutyle, cyclopentyle ou cyclohexyle (chacun facultativement substitué en position 1 avec un halogène, un groupe alkyle en C₁ à C₄ ou alkoxy en C₁ à C₄), ou le groupe R³(CH₃)₂C- dans lequel R³ représente un groupe azido, nitro, cyano, NR⁵R⁶ (dans lequel R⁵ et R⁶ représentent indépendamment H, un groupe alkyle en C₁ à C₄ ou formyle), (alkoxy en C₁ à C₄)-carbonyle ou (alkyle en C₁ à C₄)-carbonyle ; et X et Y représentent l'un et l'autre l'oxygène.

4. Composé suivant la revendication 1, dans lequel A représente un groupe fluoro, chloro, bromo, iodo ou méthyle ; B représente H, un groupe fluoro ou chloro ; D et E représentent l'un et l'autre H ; R¹ et R² représentent l'un et l'autre un groupe méthyle ; R⁴ représente un groupe 1-méthylcyclobutyle, 1-méthylcyclopentyle ou 1-méthylcyclohexyle ; et X et Y représentent l'un et l'autre l'oxygène.

5. Procédé de préparation d'un composé suivant la revendication 1, 2 ou 3, dans lequel X et Y représentent l'un et l'autre l'oxygène, qui comprend la réaction dans un solvant organique convenable en présence d'une base :
(a) d'un composé de formule (III) : 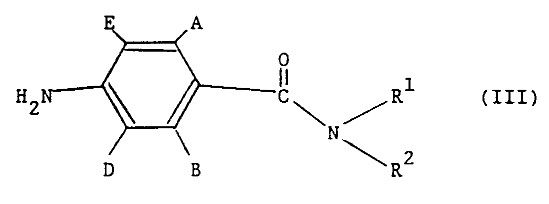 avec un chlorure d'acide de formule R⁴COCl ; ou bien
(b) d'un composé de formule (VI) : 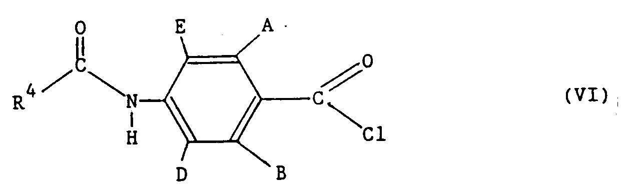 avec une amine de formule R¹R²NH ; formules dans lesquelles A, B, D, E, R¹, R² et R⁴ répondent aux définitions mentionnées dans la revendication 1, 2 ou 3.

6. Procédé de préparation d'un composé suivant la revendication 1, 2 ou 3, dans lequel X et Y représentent l'un et l'autre l'oxygène, qui comprend :
(a) lorsque R⁴ représente un groupe alkyle en C₂ à
C₈ contenant un substituant azido,
la réaction d'un composé de formule (VII) : 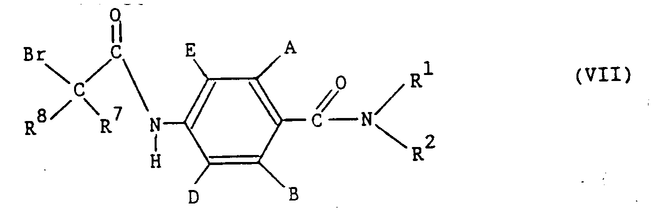 avec un azoture dans un solvant convenable, ou
(b) lorsque R⁴ représente un groupe alkyle en C₂ à C₈ contenant un substituant -NR⁵R⁶ dans lequel R⁵ et R⁶ représentent l'une et l'autre H,
la réduction d'un azoture de formule (VIII) : 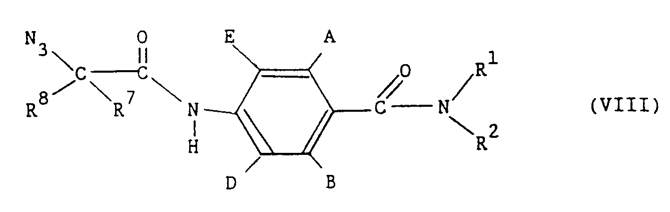
(c) lorsque R⁴ représente un groupe alkyle en C₂ à C₈ contenant un substituant -NR⁵R⁶ dans lequel R⁵ représente H et R⁶ représente un groupe formyle,
la réaction d'une amine de formule (IX) : 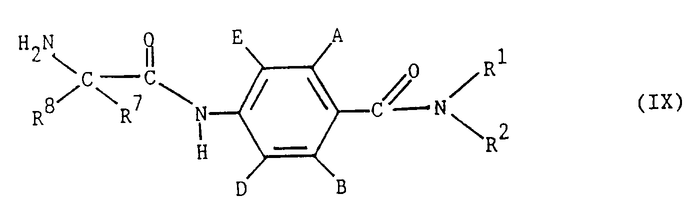 avec un mélange d'acide formique et d'un anhydride, !ou
(d) lorsque R⁴ représente un groupe cyclobutyle, cyclopentyle ou cyclohexyle contenant un substituant fluoro,
le traitement d'un alcool de formule (XI) : 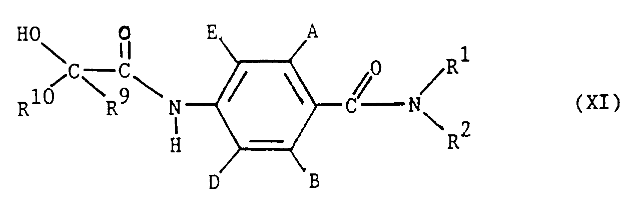 avec un agent de fluoration, ou
(e) lorsque R⁴ représente un groupe alkyle en C₂ à C₈ contenant un substituant nitro,
la réaction d'un composé de formule (VII) : 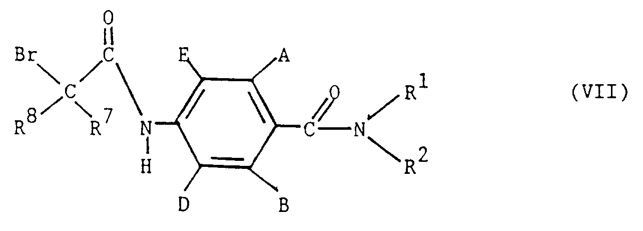 avec un nitrite métallique en présence d'urée dans un solvant convenable ; formules dans lesquelles A, B, D, E, R¹, R² et R⁴ répondent aux définitions mentionnées dans la revendication 1, le groupe R⁷R⁸C est le résidu de R⁴ lorsque R⁴ représente un groupe alkyle en C₂ à C₈ contenant un groupe bromo dans le composé de formule (VII), contenant un groupe azido dans le composé de formule (VIII), et contenant un groupe amino dans le composé de formule (IX), et R⁹R¹⁰C est le résidu de R⁴ lorsque R⁴ représente un groupe cyclobutyle, cyclopentyle ou cyclohexyle contenant un groupe hydroxy dans le composé de formule (XI).

7. Composition fongicide comprenant un quantité à effet fongicide d'un composé suivant la revendication 1, 2 ou 3 et un support ou diluant acceptable du point de vue fongicide.

8. Procédé pour combattre des champignons, qui comprend l'application à des plantes, aux semences de plantes ou au milieu dans lequel se trouvent les plantes ou les semences, d'un composé suivant la revendication 1, 2 ou 3 ou d'une composition suivant la revendication 7.
